Europäisches Patentamt

European Patent Office

Office européen des brevets

(11) Veröffentlichungsnummer: **0 238 986**
**A2**

(19)

# EUROPÄISCHE PATENTANMELDUNG

(21) Anmeldenummer: 87103921.0

(22) Anmeldetag: 18.03.87

(51) Int. Cl.⁴: **A61K 35/16 , A61K 37/02**

(30) Priorität: 25.03.86 DE 3609954

(43) Veröffentlichungstag der Anmeldung:
30.09.87 Patentblatt 87/40

(84) Benannte Vertragsstaaten:
AT BE CH DE ES FR GB GR IT LI LU NL SE

(71) Anmelder: Biotest Pharma GmbH
Flughafenstrasse 4
D-6000 Frankfurt 71(DE)

(72) Erfinder: Möller, Wolfgang, Dr. Dipl.-Chem.
Langwiesenweg 21
D-6370 Oberursel(DE)
Erfinder: Kothe, Norbert, Dr. Dipl.-Chem.
Friedrich-Ebert-Strasse 21
D-6242 Kronberg(DE)

(74) Vertreter: Beil, Hans Christoph, Dr. et al
Beil, Wolff und Beil Rechtsanwälte
Adelonstrasse 58
D-6230 Frankfurt am Main 80(DE)

(54) Verfahren zur Herstellung eines den Blutgerinnungsfaktor VIII in ankonzentrierter Form enthaltenden Präparates.

(57) Die Erfindung betrifft ein Verfahren zur Herstellung eines den Blutgerinnungsfaktor VIII in ankonzentrierter Form enthaltenden Präparates durch Behandlung einer den Blutgerinnungsfaktor VIII enthaltenden Plasmafraktion mit einer Proteinase, Abtrennung der Fibrinogenfragmente und anderer Proteine von der den Blutgerinnungsfaktor VIII enthaltenden Lösung sowie Konzentration und gegebenenfalls Gefriertrocknung dieser Lösung, bei dem man als Proteinase eine auf einem Träger immobilisierte Proteinase verwendet, diese nach Beendigung des Fibrinogenabbaus entfernt und anschließend die Fibrinogenfragmente und andere Proteine von der Lösung abtrennt.

EP 0 238 986 A2

## Verfahren zur Herstellung eines den Blutgerinnungsfaktor VIII in ankonzentrierter Form enthaltenden Präparates

Die Erfindung betrifft das in den Patentansprüchen beschriebene Verfahren zur Herstellung eines den Blutgerinnungsfaktor VIII in ankonzentrierter Form enthaltenden Präparates.

Die Gerinnungsfähigkeit gehört zu den wichtigsten Funktionen von Vollblut. Äußere oder innere Blutungen kommen bei normalem Blut innerhalb kurzer Zeit zum Stillstand. So wird der Organismus zuverlässig vor größeren Blutverlusten geschützt. Die Gerinnung läuft dabei kaskadenartig über mehrere Gerinnungsfaktoren bis hin zur Polymerisation von Fibrinogen zu Fibrin und wird enzymatisch kontrolliert.

Einer der wichtigsten Gerinnungsfaktoren ist der Blutgerinnungsfaktor VIII ( = antihaemophiles Globulin A = AHG, hier anschließend auch als Faktor VIII oder F VIII bezeichnet), der zu ungefähr 10 mg/l in normalem Plasma enthalten ist. Fehlt dieser Faktor VIII oder ist er stark vermindert, kommt es zu unkontrollierten Blutungen und damit zum Erscheinungsbild der Hämophilie A, der häufigsten angeborenen Bluterkrankheit. Die Therapie der Wahl für diese Krankheit besteht in der Substitution mit F-VIII-reichen Plasmaderivaten.

Die zur Zeit gebräuchlichsten F-VIII-Präparate sind ein durch Kälteausfällung erhaltenes Kryopräzipitat (Pool, J.G., Biblioth. Haematol.34, 23-29 (1979)), eine durch Alkoholfällung nach Cohn (J.Am.Chem.Soc. Bd. 72 (1959) 459) erhaltene Cohn-Fraktion I und F-VIII-Konzentrate mit höher angereichertem F-VIII, die überwiegend durch Präzipitationstechniken erhalten werden.

Bei der Behandlung der Hämophilie A mit Kryopräzipitat und Cohn-Fraktion I lagen die größten Nachteile in der mangelnden Reinheit der F-VIII-Präparate und der großen Volumenbelastung für die Patienten. Diese Probleme sowie die potentielle Infektiosität durch Viruskontamination wurden zwar durch die neueren, überwiegend durch Präzipitationstechniken erhaltenen F-VIII-Konzentrate zum großen Teil gelöst; aber gerade die größere Reinheit und damit höhere spezifische Aktivität der F-VIII-Konzentrate wurde mit einer wesentlich geringeren Ausbeute an Faktor VIII erkauft. Diese geringe Ausbeute wurde zusätzlich durch Sterilisationsverfahren weiter drastisch verringert.

Es besteht daher weiter ein dringender Bedarf, Faktor VIII mit hoher Reinheit aus Plasma mit wesentlich höherer Ausbeute als bisher zu isolieren, um aus dem weltweit begrenzten Plasmaaufkommen diesen Gerinnungsfaktor einerseits wirtschaftlicher zu gewinnen und damit die hohen Kosten zu senken und andererseits den immer noch nicht gedeckten Bedarf an sterilisierten, hochgereinigten F-VIII-Konzentraten zu befriedigen.

Das größte Problem bei der Reinigung von Faktor VIII besteht in der Abtrennung von Fibrinogen. Größere Mengen gerinnbares Fibrinogen in F-VIII-Konzentraten führen zu einer schlechten Löslichkeit dieser Präparate und nach wiederholter Applikation zu den Nebenwirkungen, die durch eine Akkumulation von Fibrinogen im Organismus hervorgerufen werden.

Die in der Praxis durchgeführten Verfahren zur Trennung von Faktor VIII und Fibrinogen bedienen sich der Fällung von Fibrinogen und/oder Faktor VIII mit Ethanol, Ether, Polyethylenglykol (PEG), Ammoniumsulfat, Glycerin, NaCl oder anderen Fällungsreagenzien sowie der unterschiedlichen Löslichkeit bei verschiedenen pH-Werten bzw. Temperaturen in wäßgen Pufferlösungen. Wegen der ähnlichen physikalischen und chemischen Eigenschaften von Fibrinogen und Faktor VIII führen diese Standardverfahren sowiee chromatographische Methoden oder Elektrophorese zu keiner scharfen Trennung der beiden Proteine ohne große Ausbeuteverluste von Faktor VIII.

Eine Möglichkeit zur Veränderung der physikalischen und chemischen Eigenschaften von Proteinen und damit eine Möglichkeit zu ihrer Trennung besteht in ihrer Proteolyse. Fibrinogen und Faktor VIII reagiern unterschiedlich mit Proteinasen. Faktor VIII ist normalerweise sehr empfindlich gegenüber einem proteolytischen Angriff. Er wird schon durch geringe Mengen von Proteinasen inaktiviert, wobei die Inaktivierung mit einer vorhergehenden Aktivierung verbunden sein kann (M.E.Switzer et al., J. Biol. Chem. 255 (22), 1980, 10606-10611; V. Atichartakarn et al., Blood 51 (2), 1978, 281-297). Fibrinogen wird z.B. durch Plasmin, Trypsin oder Chymotrypsin in mehr oder weniger große Bruchstücke zerlegt, während Thrombin durch Spaltung einer Bindung im Fibrinogen dieses zu Fibrin polymerisiert.

Eine von E.M. Barrow und Mitarbeitern (Proc. of the Soc. for Experim. Biol. and Med. 121 (1966), S.1001-1005) beschriebene Methode nutzt diese Reaktion von Thrombin mit Fibrinogen zur Trennung des Faktors VIII vom Fibrinogen. Während normalerweise Faktor VIII mit Thrombin sofort aktiviert und anschließend inaktiviert wird, sollen $Mn^{2+}$-Ionen den Faktor VIII so schützen, daß er in seiner Aktivität erhalten bleibt. Neben Ausbeuteverlusten von Faktor VIII, die durch Einschluß in das Fibringerinnsel entstehen, liegt der größte Nachteil dieser Methode in dem hohen Mn²⁺-Gehalt solcher F-VIII-Präparationen und den damit verbundenen, an Hunden beobachteten Vergiftungserscheinungen, was einen Einsatz als Therapieprodukt ausschließt.

Ch.N.Vogel und Mitarbeiter (Thrombos. Diathes. haemorrh. 1973, 30, 229) filtrierten eine vorher durch Fällung mit Aminosäuren von Fibrinogen gereinigte F-VIII-Lösung durch eine Trypsin-Agarose-Säule und veränderten den Faktor VIII in seinem Molekulargewicht und seiner Aktivierbarkeit. Abgesehen von dem bei diesem Verfahren auftretenden Abbau des Faktors VIII eignet sich dieses Verfahren auch nicht zur Trennung von Fibrinogen und Faktor VIII, da durch Trypsin zuerst Faktor VIII und dann erst Fibrinogen abgebaut wird.

S.L. Marchesi und Mitarbeiter (The Journal of Clinical Investigation 51, 1972, S. 2151-2161) beschreiben die Präparation von Faktor VIII für analytische Zwecke durch Abbau von Fibrinogen mit gelöstem Chymotrypsin und anschließende Gelfiltration über Agarose (Sepharose 4 B^(R)). Diese Methode hat mehrere Nachteile, die ihren Einsatz zur Gewinnung eines Therapieproduktes von Faktor VIII in großem Maßstab unmöglich machen. So ist der Faktor VIII nach der Abrennung von den Fibrinogenbruchstücken instabil und kann ohne Stabilisatoren nur mit einer Ausbeute von 7 %, bezogen auf eingesetzten Faktor VIII, isoliert werden. Nach der Gefriertrocknung ist überhaupt keine F-VIII-Aktivität mehr nachweisbar. Diese Instabilität von mit gelösten Proteinasen behandeltem Faktor VIII wird auch von anderen Autoren beschrieben (V. Atichartakarn et al., Blood 51 (1978), 281-297).

Der Fibrinogenabbau selbst muß durch schrittweise Zugabe von Chymotrypsin vorsichtig austitriert werden, um F-VIII-Verluste bei der Proteolyse zu vermeiden und um das Ende des Fibrinogenabbaus zu ermitteln. Die nächste Schwierigkeit ist dann die sofortige Abtrennung der Proteinase nach Beendigung der Reaktion.

Die von S.L. Marchesi und Mitarbeitern angewandte Agarose-Gelfiltration ist in den vorgestellten Dimensionen für eine großtechnische Durchführung nicht wirtschaftlich, abgesehen davon, daß eine damit im kleinen Laboransatz durchfuhrbare schnelle Abtrennung des Chymotrypsins in großem Maßstab mit Ansatzgrößen von mindestens 100 l an den zwangsläufigen, technisch bedingten Standzeiten scheitern muß. Somit ist dies kein praktikabler Weg zur großtechnischen Gewinnung eines F-VIII-Konzentrates zur Therapie der Hämophilie A.

Der Erfindung liegt die Aufgabe zugrunde, ein Verfahren zur Herstellung eines den Blutgerinnungsfaktor VIII in ankonzentrierter Form enthaltenden Präparates durch Behandlung einer den Blutgerinnungsfaktor VIII enthaltenden Plasmafraktion mit einer Proteinase, Abtrennung der Fibrinogenfragmente und anderer Proteine von der den Blutgerinnungsfaktor VIII enthaltenden Lösung sowie Konzentration und gegebenenfalls Gefriertrocknung dieser Lösung bereitzustellen, bei dem unter Vermeidung der bisherigen großen Ausbeuteverluste bei der Abtrennung von Fibrinogen und anderen störenden Proteinen vom Faktor VIII das Fibrinogen so in seinen physikalischen und chemischen Eigenschaften verändert wird, daß es leicht vom Faktor VIII abgetrennt werden kann.

Diese Aufgabe wird erfindungsgemäß dadurch gelöst, daß man als Proteinase eine auf einem Träger immobilisierte Proteinase verwendet, diese nach Beendigung des Fibrinogenabbaus entfernt und anschließend die Fibrinogenfragmente und andere Proteine von der Lösung abtrennt.

Nach dem erfindungsgemäßen Verfahren wird ein für die Therapie der Hämophilie A geeignetes F-VIII-Konzentrat mit einer spezifischen Aktivität von mindestens 2 Einheiten Faktor VIII pro mg Protein und einer Konzentration von mindestens 25 Einheiten Faktor VIII pro ml erhalten, das höchstens geringe, die Therapie nicht beeinträchtigende Mengen Fibrinogen enthält.

Überraschenderweise wurde gefunden, daß der Vorteil der Verwendung der immobilisierten Proteinase nicht einer schnelleren Abtrennung der Proteinase aus nur in einer schnelleren Abtrennung der Proteinase aus dem Reaktionsgemisch liegt, sondern daß auf diese Weise auch der Abbau des Fibrinogens so schonend erfolgt, daß der Faktor VIII nicht beeinträchtigt wird, was sich z.B. darin äußert, daß auch ohne Stabilisator seine Aktivität bis nach der Gefriertrocknung erhalten bleibt.

Dieser Unterschied in der Stabilität zu solchen F-VIII-Präparaten, die mit gelöstem Chymotrypsin hergestellt wurden, zeigt sich auch in der erfindungsgemäß ohne Ausbeuteverluste durchfuhrbaren Ultra-und Diafiltration. S.L. Marchesi und Mitarbeiter berichten dagegen uber eine große Instabilität ihres mit gelöstem Chymotrypsin hergestellten Faktors VIII nach der Konzentrierung durch Ultrafiltration.

Bei der Durchführung des erfindungsgemäßen Verfahrens wird zunächst eine den Faktor VIII enthaltende Plasmafraktion, z.B. ein Kryopräzipitat, eine Cohn-Fraktion I oder eine durch andere Fällungsverfahren erhalten Fraktion mit einem Gehalt an Faktor VIII, in einer Pufferlösung gelöst. Hierzu eignen sich Pufferlösungen mit niedriger oder hoher Ionenstärke, deren pH-Wert zwischen 5,5 und 8,5, vorzugsweise zwischen 6,5 und 8 liegt. Das Lösen kann bei Temperaturen von 25 bis 37°C durchgefuhrt werden.

Sodann wird die Lösung der F-VIII-haltigen Plasmafraktion mit einer auf einem Träger immobilisierten Proteinase versetzt. Die Proteinase soll Fibrinogen weitgehend abbauen, bevor Faktor VIII proteolytisch angegriffen wird. Dies wird vorzugsweise mit Proteinasen mit einer Spezifität für aromatische und hydrophobe Aminosäuren, z.B. Chymotrypsin, Pepsin oder Thermolysin, erreicht.

Die erfindungsgemäß verwendete Proteinase ist auf einem Träger immobilisiert. Trägermaterialien für diese Immobilisierung sind bekannt. Beispiele für derartige Trägermaterialien sind Acrylamidpolymere, insbesondere ein Copolymerisat aus Methacrylamid und N-Methylen-bis-methacrylamid, Agarose, Cellulose, Dextran, Agar, Silikate und Glas. Die Immobilisierung der Proteinase mit diesen Trägermaterialien wird nach bekannten Verfahren durchgeführt, wie sie z.B. in "Methods in Enzymology", Bd. 44, S.19ff beschrieben sind. Die Trägermaterialien werden zunächst mit verschiedenen reaktiven Gruppen aktiviert und sodann an die Proteinase gebunden. Acrylamidpolymere können beispielsweise nach einer Aktivierung mit Oxirangruppen oder nach einer Glutardialdehyd-, Bromcyan-oder Carbodiimidaktivierung zur Immobilisierung der Proteinase über kovalente Bindungen verwendet werden. Auch die direkte kovalente Kopplung der Proteinase an Hydroxylgruppen von Agarose, Cellulose, Dextran oder Agar über reaktive Gruppen wie die Imidocarbonat-, Oxiran-oder Vinylketogruppe oder über aktiviertes Halogen sowie die indirekte Bindung an Spacer mit Carboxyl-, Amino-oder Sulfhydrylgruppen kann angewendet werden. Aktivierte Trägermaterialien, z.B. ein mit Oxirangruppen aktiviertes Copolymerisat aus Methacrylamid und N-Methylen-bis-methacrylamid, eine mit Bromcyan aktivierte Agarose oder ein mit Glutardialdehyd aktiviertes Silikat, sind im Handel erhältlich, und die Hersteller Röhm Pharma GmbH, Pharmacia Fine Chemicals bzw. Boehringer Mannheim GmbH geben für diese Trägermaterialien, d.h. Eupergit C[(R)], Sepharose 6MB[(R)] bzw. Affinitätsadsorbens, in ihren Handelsbroschüren die Immobilisierungsverfahren an.

Die Umsetzung der gelösten F-VIII-haltigen Plasmafraktion mit der immobilisierten Proteinase wird bei einer Temperatur von 4 bis 40°C, vorzugsweise 20 bis 25°C, durchgeführt. Die Menge der immobilisierten Proteinase wird dabei so bemessen, daß der Abbau des Fibrinogens in 10 Minuten bis 24 Stunden, vorzugsweise in 3 bis 5 Stunden, beendet ist. Dies erreicht man mit 1 bis 50 ml, vorzugsweise 5 bis 10 ml, der feuchten immobilisierten Proteinase pro g Fibrinogen. Eine kürzere Reaktionszeit läßt weniger Zeit für die Abtrennung der Proteinase vor der Proteolyse des Faktors VIII. Eine längere Reaktionsdauer bringt eventuell Probleme mit der Pyrogenität durch Keimwachstum in der Reaktionslösung. Nach Beendigung des Fibrinogenabbaus kann die immobilisierte Proteinase sofort, z.B. durch Filtration oder Zentrifugation, abgetrennt werden. Ebenso ist die kontinuierliche Überströmung der unlöslichen Proteinase und Weiterverwendung des Eluats möglich. Durch den erfindungsgemäßen Einsatz einer immobilisierten Proteinase wird auf diese Weise die sofortige Beendigung der proteolytischen Reaktion auch im großtechnischen Maßstab praktikabel.

Nach der Entfernung der immobilisierten Proteinase aus der Lösung werden die Fibrinogenfragmente, anderen Begleitproteine sowie niedermolekulare Bestandteile von der Lösung, die gegebenenfalls vorher noch geklärt und/oder kaltsterilisiert werden kann, abgetrennt. Diese Abtrennung erfolgt vorzugsweise durch Diafiltration über eine Membran mit der Ausschlußgrenze MG 100 000 bis 2 000 000 D, insbesondere MG 300 000 bis 1 000 000 D. Da das abgebaute Fibrinogen weitgehend ungerinnbar ist, kommt es dabei nicht zu den bei der Diafiltration von Kryopräzipitatlösung zu beobachtenden negativen Erscheinungen, wie Verstopfen der Membranporen (und damit verbundener niedriger Flußrate) sowie Adsorption von Faktor VIII an Fibrin/Fibrinogen auf der Membraninnenseite.

Nach der Abtrennung der Fibrinogenfragmente und Begleitproteine und gegebenenfalls einer Kaltsterilisation, z.B. durch Behandlung mit β-Propiolacton und/oder UV-Strahlen, zwecks Sicherstellung der Virusfreiheit wird die den Faktor VIII enthaltende Lösung auf eine Konzentration von mindestens 25 Einheiten Faktor VIII pro ml konzentriert. Diese Konzentration kann mit der gleichen Anlage wie die Abtrennung der Fibrinogenfragmente als Ultrafiltration oder nach anderen bekannten Verfahren durchgeführt werden.

Anschließend kann die konzentrierte Lösung sterilfiltriert und gefriergetrocknet werden. Die Sterilfiltration und die Gefriertrocknung erfolgen in bekannter Weise und dienen der Sicherstellung eines stabilen, sterilen und pyrogenfreien Therapieproduktes.

Gemäß einer bevorzugten Ausführungsform der Erfindung wird ein Kryopräzipitat in einer Pufferlösung gelöst und bei 20 bis 25°C mit 5 bis 10 ml eines feuchten, durch kovalente Bindung an ein Copolymerisat aus Methacrylamid und N-Methylen-bis-methacrylamid immobilisierten Chymotrypsin pro g Fibrinogen im Kryopräzipitat umgesetzt, so daß der Abbau des Fibrinogens nach etwa 3 bis 5 Stunden beendet ist. Anschließend wird das immobilisierte Chymotrypsin möglichst schnell durch Filtration, z.B. durch ein Metallsieb, abgetrennt. Die resultierende Proteinlösung wird sodann zwecks Klärung und/oder Sterilfiltration einer Membranfiltration über eine Membran von 0,1 bis 5 μm,

vorzugsweise von 0,2 μm, unterworfen. Anstelle der Membranfiltration kann auch eine Tangentialfiltration an einer Hohlfaser-oder Flachmembran von 0,1 bis 5 μm, vorzugsweise von 0,2 μm, durchgeführt werden. Anschließend wird das geklärte Filtrat über eine Membran mit der Ausschlußgrenze MG 300 000 bis 1 000 000 D diafiltriert, um die Fibrinogenfragmente und Begleitproteine zu entfernen. Schließlich wird das Retentat der Diafiltration auf eine Konzentration von mindestens 25 Einheiten Faktor VIII pro ml konzentriert und nach einer Sterilfiltration gefriergetrocknet. Vorzugsweise wird nach der Membran-oder Tangentialfiltration oder insbesondere nach der Diafiltration eine Kaltsterilisation durch Behandlung mit β-Propiolacton und UV-Strahlen durchgeführt.

Nach dem erfindungsgemäßen Verfahren wird ein F-VIII-Konzentrat mit hoher spezifischer Aktivität von beispielsweie 25 Einheiten Faktor VIII pro mg Protein und mit weniger als 100 mg gerinnbarem Fibrinogen pro 1000 Einheiten Faktor VIII erhalten. Eine Präparation nach diesem Verfahren zeigt eine sehr gute Löslichkeit nach der Gefriertrocknung und läßt sich auch zur Sicherstellung einer Virusinaktivierung sterilisieren, z.B. mit β-Propiolacton und UV-Licht.

Die folgenden Beispiele erläutern die Erfindung.

Beispiel 1:

9 Teile Spenderblut wurden zu 1 Teil 3,8 %iger Natriumcitrat-Stabilisatorlösung gegeben. Das Blut wurde sofort nach der Blutabnahme zentrifugiert und das Plasma möglichst bald auf -40°C eingefroren.

Aus 100 l gefrorenem Plasma wurde in bekannter Weise durch Auftauen auf 4°C und anschließende Zentrifugation das Kryopräzipitat vom Restplasma abgetrennt.

Die angefallenen 800 g Kryopräzipitat wurden in 3200 ml Glycinpuffer vom pH 7,4 (Puffer I) gelöst. Anschließend wurde ein mit einem Copolymerisat aus Methacrylamid und N-Methylen-bis-methacrylamid immobilisiertes Chymotrypsin zugesetzt, das wie folgt hergestellt worden war:

20 g Chymotrypsin (Sigma Nr. C 4129) wurden in 500 ml 1 M Kaliumphosphatpuffer vom pH 7,0, der 0,05 % para-Hydroxybenzoesäureethylester enthielt, gelöst, mit 130 g eines mit Oxirangruppen aktivierten Copolymerisates aus Methacrylamid und N-Methylen-bis-methacrylamid (Eupergit C[(R)] der Röhm Pharma GmbH) versetzt und 72 Stunden bei Raumtemperatur stehengelassen. Anschließend wurde mit 20 l 0,1 M Kaliumphosphatpuffer vom pH 7,0 gewaschen.

Die Lösung des Kryopräzipitats mit dem immobilisierten Chymotrypsin wurde bei 25°C 3,5 Stunden gerührt, bis kein gerinnbares Fibrinogen mehr nachweisbar war.

Die Gerinnungsfähigkeit des Fibrinogens im Reaktionsgemisch wurde von Zeit zu Zeit durch Zugabe von 1 Teil Thrombinlösung (60 Einheiten pro ml) und 0,5 Teilen 0,025 M CaCl$_2$ zu 1 Teil Probe ermittelt. Wenn nach 5 min bei 37°C kein Fibringerinnsel zu sehen war, wurde die Proteolyse beendet.

Das immobilisierte Chymotrypsin wurde sofort durch Filtration abgetrennt und mit Puffer I nachgewaschen.

Das Filtrat (4500 ml) wurde über ein 0,2 μm-Membranfilter filtriert und anschließend über eine Membran mit einer Ausschlußgrenze von MG 300 000 D diafiltriert. Die Diafiltration wurde zu Beginn mit Puffer I und dann mit dem für die Gefriertrocknung vorgesehenen physiologisch verträglichen Glycinpuffer vom pH 7,2 durchgeführt. Das Retentat, das den Faktor VIII enthielt, wurde durch Ultrafiltration ankonzentriert und auf eine F-VIII-Konzentration von über 25 Einheiten pro ml eingestellt. Nach einer Sterilfiltration über ein 0,2 μm-Membranfilter wurde das Produkt gefriergetrocknet.

Bei einer spezifischen Aktivität von über 3 Einheiten Faktor VIII pro mg Protein war die Ausbeute mehr als doppelt so hoch wie bei herkömmlichen Reinigungsverfahren zu F-VIII-Konzentraten mit vergleichbarer Reinheit.

Die F-VIII-Gerinnungsaktivität wurde mit der Einstufenmethode nach dem Normentwurf DIN 58909, Teil 1, bestimmt.

Das Gesamtprotein wurde mit der Biuret-Methode (Reinhold, J.G., Standard Methods of Clinical Chemistry 1, 88, (1953)) und nach Kjeldahl (Hiller, A. u. Mitarbeiter, J. Biol. Chem. 176, (1948), 1401) bestimmt.

Beispiel 2:

Die Präparation wurde in gleicher Weise wie in Beispiel 1 durchgeführt, jedoch wurde anstelle des immobilisierten Chymotrypsins nach der gleichen Methode mit einem Copolymerisat aus Methacrylamid und N-Methylen-bis-methacrylamid immobilisiertes Pepsin eingesetzt. Das Endprodukt hatte eine vergleichbare Reinheit zu dem gemäß Beispiel 1 erhaltenen F-VIII-Konzentrat, während die Ausbeute um etwa 20 % darunter lag.

Beispiel 3:

Die Präparation wurde in gleicher Weise wie in Beispiel 1 durchgeführt, jedoch wurde anstelle des immobilisierten Chymotrypsins nach der gleichen Methode mit einem Copolymerisat aus Methacrylamid und N-Methylen-bis-methacrylamid immobilisiertes Thermolysin eingesetzt.

Die Ergebnisse waren vergleichbar mit dem Beispiel 2. Eine gleich hohe Reinheit stand einer um etwa 10 % geringeren Ausbeute gegenüber.

Beispiel 4:

Die Präparation wurde in gleicher Weise wie in Beispiel 1 durchgeführt, jedoch wurde das Chymotrypsin an BrCN-aktivierte Agarose (Sepharose 4B⁻ (R)) gebunden und analog zu Beispiel 1 eingesetzt.

Die Reinheit des F-VIII-Konzentrates war mit 4,5 Einheiten Faktor VIII pro mg Protein größer als in Beispiel 1, während die Ausbeute rund 20 % niedriger, aber immer noch weit über der mit den herkömmlichen Verfahren erreichbaren Ausbeute lag.

Beispiel 5:

Die Präparation wurde in gleicher Weise wie in Beispiel 4 durchgeführt, jedoch wurde das Chymotrypsin an Glutardialdehyd-aktiviertes · Silikat (Affinitätsadsorbens von Boehringer Mannheim GmbH) gebunden.

Die Reinheit und Ausbeute waren vergleichbar mit Beispiel 1.

Beispiel 6:

1 l in gleicher Weise wie in Beispiel 1 gewonnenes Plasma wurde nach der Präparationsmethode von Cohn (J. am. Chem. Soc., Bd. 72 (1959) 465) fraktioniert. Die Fraktion I wurde mit einer Glycinlösung gewaschen und der zurückgebliebene Niederschlag in 300 ml Glycinpuffer gelöst. Anschließend wurde in gleicher Weise wie in Beispiel 1 weiterverfahren.

Die Ausbeute und die Reinheit des Faktor-VIII-Konzentrates war mit der des Beispiels 1 vergleichbar.

**Ansprüche**

1. Verfahren zur Herstellung eines den Blutgerinnungsfaktor VIII in ankonzentrierter Form enthaltenden Präparates durch Behandlung einer den Blutgerinnungsfaktor VIII enthaltenden Plasmafraktion mit einer Proteinase, Abtrennung der Fibrinogenfragmente und anderer Proteine von der den Blutgerinnungsfaktor VIII enthaltenden Lösung sowie Konzentration und gegebenenfalls Gefriertrocknung dieser Lösung, dadurch gekennzeichnet, daß man als Proteinase eine auf einem Träger immobilisierte Proteinase verwendet, diese nach Beendigung des Fibrinogenabbaus entfernt und anschließend die Fibrinogenfragmente und andere Proteine von der Lösung abtrennt.

2. Verfahren gemäß Anspruch 1, dadurch gekennzeichnet, daß man als den Blutgerinnungsfaktor VIII enthaltende Plasmafraktion ein Kryopräzipitat, eine Cohn-Fraktion I oder eine durch andere Fällungsverfahren erhaltene Fraktion mit einem Gehalt an Blutgerinnungsfaktor VIII verwendet.

3. Verfahren gemäß Anspruch 1 oder 2, dadurch gekennzeichnet, daß man als Proteinase eine solche mit einer Spezifität für aromatische und hydrophobe Aminosäuren verwendet.

4. Verfahren gemäß Anspruch 3, dadurch gekennzeichnet, daß man als Proteinase Chymotrypsin, Pepsin oder Thermolysin verwendet.

5. Verfahren gemäß einem der Ansprüche 1 bis 4, dadurch gekennzeichnet, daß man als immobilisierte Proteinase eine auf einem Copolymerisat aus Methacrylamid und N-Methylen-bis-methacrylamid immobilisierte Proteinase verwendet.

6. Verfahren gemäß einem der Ansprüche 1 bis 4, dadurch gekennzeichnet, daß man als immobilisierte Proteinase eine auf einer Agarosematrix immobilisierte Proteinase verwendet.

7. Verfahren gemäß einem der Ansprüche 1 bis 4, dadurch gekennzeichnet, daß man als immobilisierte Proteinase eine auf einer Silikatmatrix immobilisierte Proteinase verwendet.

8. Verfahren gemäß einem der Ansprüche 1 bis 7, dadurch gekennzeichnet, daß man die Fibrinogenfragmente und anderen Proteine durch Diafiltration über eine Membran mit der Ausschlußgrenze MG 100 000 bis 2 000 000 D, vorzugsweise MG 300 000 bis 1 000 000 D, von der den Blutgerinnungsfaktor VIII enthaltenden Lösung abtrennt.